(19) Europäisches Patentamt
European Patent Office
Office européen des brevets

(11) **EP 0 466 872 B1**

(12) **EUROPEAN PATENT SPECIFICATION**

(45) Date of publication and mention
of the grant of the patent:
**08.04.1998 Bulletin 1998/15**

(21) Application number: **91903102.1**

(22) Date of filing: **09.01.1991**

(51) Int. Cl.⁶: **A61B 17/38**

(86) International application number:
**PCT/US91/00024**

(87) International publication number:
**WO 91/10405 (25.07.1991 Gazette 1991/17)**

(54) **THERMAL ATHERECTOMY DEVICE**

**THERMISCHE ATHEREKTOMIEVORRICHTUNG**

**DISPOSITF D'ATHERECTOMIE THERMIQUE**

(84) Designated Contracting States:
**AT BE CH DE DK ES FR GB IT LI LU NL SE**

(30) Priority: **12.01.1990 US 464399**
**05.10.1990 US 593790**

(43) Date of publication of application:
**22.01.1992 Bulletin 1992/04**

(73) Proprietor: **METCAL INC.**
**Menlo Park California 94025 (US)**

(72) Inventors:
• **TAYLOR, James, M.**
**Mountain View, CA 94040 (US)**
• **GAY, Eric, L.**
**St. Paul, MN 55133-3427 (US)**
• **COWELL, Mark, J.**
**San Carlos, CA 94070 (US)**
• **WIRT, David, F.**
**St. Paul, MN 55133-3427 (US)**

(74) Representative:
**Hayward, Denis Edward Peter et al**
**Lloyd Wise, Tregear & Co.,**
**Commonwealth House,**
**1-19 New Oxford Street**
**London WC1A 1LW (GB)**

(56) References cited:
US-A- 4 654 024          US-A- 4 745 264
US-A- 4 760 845          US-A- 4 769 519
US-A- 4 790 311          US-A- 4 807 620
US-A- 4 978 346

## Description

The present invention relates to catheters and the like and more particularly to steerable, heated catheters for removing atherosclerotic plaque from coronary as well as peripheral arteries.

Atherosclerotic plaque is a relatively common occurrence in these times of rich foods and long life. The plaque produces a stenosis reducing the diameter of the lumen of the artery and restricting blood flow to the region beyond the stenosis. In some instances a balloon catheter may be employed to increase the diameter of the lumen particularly where complete blockage of the artery has not occurred. The balloon increases the diameter of the vessel by stretching the wall beyond the limit of elastic recovery but does not compress plaque that has hardened. In those instances where a balloon catheter cannot be used or cannot be used initially, catheters may be used to bore through the plaque and increase the diameter of the lumen through the stenosis so that if necessary a balloon catheter may be employed.

The use of steerable catheters to remove atherosclerotic plaque both from coronary as well as peripheral arteries is in increasing use today. In the art today, there are heated catheters, cutting blade catheters and various types of laser catheters all of which may be quite dangerous in use since, if aimed incorrectly or overheated, they can damage the wall of an artery producing serious, if not fatal, injury or cause particles to enter the blood stream.

Various approaches to reducing these hazards have been suggested. Very thin guide wires may be employed to facilitate guiding the catheter to the proper location. Although probably not used in practice, some patents disclose feeding a laser through the arteries to remove plaque. To better control various factors such as size of the instrument, aim of the beam and the energy supplied by the laser, optical fibers are now employed to conduct laser energy to the site of the stenosis.

Various of these techniques are being employed today but dangers of overheating and misdirection are still prevalent although the use of guide wires has materially reduced the danger of mechanical puncture of the wall of an artery. Further, there is still the danger that solid debris from the plaque or liquified plaque may enter the blood stream and produce serious blockage, particularly in small coronary arteries.

The use of a heated catheter requires a delicate balance of various factors. It is desirable to concentrate the heat in an axially forward direction at the distal face of the heater of the catheter while maintaining the sides of the catheter at a relatively low temperature. The distal face of the catheter must be able to rapidly reach a temperature that produces vaporization of the plaque. As to the range of allowable temperatures of the sidewall of the catheter, the upper end of the range must be below a temperature at which the catheter will stick to the wall of the artery, or approximately 120°C. Such temperature, however, should be high enough to damage without charring the inner surface cells of the wall so that the rate of future adherence of plaque to the wall will be greatly reduced.

Such control of sidewall temperature of a heated catheter can be achieved by maximizing forward heat flow while carefully controlling flow of heat from the distal face of the heater towards its proximal end and radially from the core of the heater to the sidewall whereby the length of the sidewall behind the head that is at temperatures that can produce sticking is sufficiently short as to not allow sticking during use.

US Patent No. 4807620 describes a thermal angioplasty apparatus according to the preamble of claim 1 which includes a probe comprising a transmission line such as a coaxial cable and an inductive load such as a ferrite bead at the remote or distal end of the line. The power applied to the proximal end of the line and that reflected from the distal end are measured to allow the former to be adjusted to control the distal end temperature. In the illustrated embodiment at the line distal end the central conductor of the coaxial cable is surrounded by an inner insulative layer and an outer conducting layer of e.g. wire mesh which in turn is covered by an insulative coating. The central conductor extends beyond the layers through a ferrite bead and into a metallic cap which electrically connects the centre conductor and the outer conducting layer.

US Patent 4769519 describes a ferromagnetic element for use, eg. as a soldering iron through which radio frequency current is passed directly or by induction. The current amplitude is chosen to heat the element well above its Curie temperature. Permeability drop-off at the Curie temperature is sensed and used to cut the current supply so regulating the temperature of the element at or near the Curie temperature.

It is amongst the objects of the present invention to provide a small, temperature self regulating, heated catheter for use in removing plaque from arteries of humans and animals, which catheter may be employed with a guide wire and which minimizes danger to the walls of the arteries.

A thermal atherectomy device for use in blood vessels and the like in accordance with the invention comprises a tip including a cylindrical body terminating at one end in a head, characterised in that the tip is of high magnetic permeability, in that the head is enlarged and the cylindrical body terminates at its other end in an enlarged collar, and, in that the device includes a coil of wire wound about the cylindrical body essentially abutting the head and removed from the collar and means for connecting the coil to a source of alternating current.

The thermal atherectomy device may be included in an interventional therapeutic device for remote delivery of heat to body tissue which comprises an oscillator for providing an r.f. output signal within the range of 10 MHz to 2 GHZ. A transmission line has a proximal end connected to the oscillator for receiving the output signal and a distal end, the transmission line being constructed and arranged to pass through the interior of a body cavity. The coil and tip comprise an inductive load disposed at the distal end of the transmission line, the coil operating to convert the r.f. signal transmitted through the transmission line into heating of the tip. The conversion is preferably optimized at a predetermined frequency, the frequency of the output signal from the oscillator being set at substantially the predetermined frequency, whereby the tip is heated to a temperature sufficient for therapeutic effects.

In a preferred embodiment, the device is a catheter, with the tip at its distal end, for use in removing atherosclerotic plaque in coronary and peripheral arteries. Preferably the catheter operates in conjunction with a guide wire so as to be guided along an artery to the location of a stenosis. A fine guide wire can be more readily manipulated than a catheter and, as such, greatly reduces the danger of entering a wrong artery or puncturing the wall of an artery. The catheter can be easily slid along the guide wire to the desired location.

The use of the guide wire in conjunction with a temperature self regulating heater removes the most common dangers in the use of such instruments. The use of the guide wire is not essential but when the catheter is constructed such as to be usable with a wire, the margin of safety is greatly increased.

The tip provides a temperature self regulating heater at the distal end of the catheter. Temperature regulation is accomplished by employing a material having a high magnetic permeability such as a ferromagnetic, ferrimagnetic or the like material having a Curie temperature at the maximum temperature of heater operation. As pointed out in U.S. Patent No. 4,914,267 a ferromagnetic conductor connected in series in a circuit having a high frequency, constant current supply or subject to a varying magnetic field at such high frequency regulates temperature up to and about the effective Curie temperature of the ferromagnetic material. The effective Curie temperature is that at which the material becomes sufficiently close to paramagnetic for purposes of operation of the heater in the present environment. Such temperature may however be a few or as much as 100 degrees less than absolute Curie temperature where the material becomes truly paramagnetic.

The high mu material increases in temperature until the effective Curie temperature is approached. As this occurs the resistance of the heater decreases and, since current is constant (K), the power dissipation is controlled by the equation: $P = KR$. The reduction in resistance causes a reduction of power dissipation. The heater stabilizes at an equilibrium temperature which is maintained until the load on the heater changes and the heating cycle is repeated until equilibrium is again established.

In a magnetic flux coupled heater the high mu material is subject to a high concentration of magnetic flux causing the material to heat. Upon approaching Curie temperature, the material becomes generally paramagnetic or the permeability is so reduced that the flux coupling is greatly reduced and the heater cools until an equilibrium condition is again established.

The temperature of the device at the stenosis must be such as to ablate the plaque by vaporization, thus preventing pieces of the plaque or melted plaque from entering the blood stream. Also the design of the structure is preferably such that at such a temperature only a desired degree of damage is done to the walls of the artery. It is desirable to damage, without charring, the cells of the inner surface of the artery so as to reduce the rate of buildup of plaque in the future.

The Curie temperature of the high mu material is the maximum temperature of the device which, in practice, is not often achieved but is approached when the stenosis is engaged and blood is essentially excluded from the region of contact. The operating temperature range of the various Curie temperature materials employed in conjunction with the structure described below is a function of the environment in which employed. It is essential that the temperature of the distal face contacting the plaque be above 300°C. It has been found that at a heater Curie temperature of 400°C the catheter will tend to stick to the walls of the vessels. Thus materials having Curie temperatures of 450°C to approximately 620°C are desired and have been found satisfactory, the latter permitting a more rapid procedure, decreasing the margin of thermal damage to the walls of the arteries by decreasing the transit time through the stenosis. The Curie temperature selected provides the utility and safety of a rapid procedure with only a quite short length of sidewall at temperatures that can produce sticking, that is, temperatures of 120°C or more.

As discussed above the catheter is preferably movable along a guide wire for purposes of removing plaque from coronary and peripheral arteries and is thus a thermal atherectomy catheter. The size of the catheter described herein is employable in distal peripheral and the coronary arteries. The heater comprises a hollow, cylindrical body of, for instance, a ferromagnetic material having a flared head at one end, the distal end, of the cylinder. A collar is located adjacent the other, proximate, end of the hollow cylindrical heater and insulated wire is wound helically in several layers about the cylindrical body between the head and collar. In a first embodiment various coatings are applied over the wire until a smooth transition exists between the head and collar. In this embodiment the maximum diameter of the catheter is at the location of the collar. The maximum diameter of the device varies with intended use and typically in conjunction with peripheral arteries may vary from 1 to 4 mm (.040 to .160 inch).

In a second embodiment the wire (coil) is potted in a thermally conductive electrically insulative material and a thermally conductive ferromagnetic metal tube or sleeve of lesser diameter than the head surrounds the potted coil. A further metal tube or sleeve of the diameter of the head extends from the head to the collar providing an insulating space between the two sleeves. This space may be filled, for instance, by microshells fused to one another to provide a complete filler which is 95% to 98% air. In this embodiment the collar is integrated with the cylindrical body.

The inner metal sleeve is a 400 series stainless steel which is ferromagnetic while the outer sleeve is a 300 series stainless steel which is non-magnetic. The preferred materials are stainless 430 and 316, respectively. The coil is not as long as the space between the head and collar and is disposed immediately adjacent the head so that the magnetic flux produced by the coil is coupled directly to the head as well as the cylindrical body and inner sleeve while having little coupling to the collar. In consequence of this arrangement, that is, the coil remote from the collar with magnetic flux and heat coupled directly to the head as well as the cylindrical body and inner sleeve, the insulating space between the inner and outer sleeve and potting with a heat conductive material, the heat is concentrated in the head. Also the ferromagnetic sleeve of 430 stainless together with the non-ferromagnetic outer sleeve concentrates the flux radially inward of the catheter thus increasing efficiency and reducing radiation of magnetic flux into surrounding tissue. The only region of the device that both develops high temperature and radially contacts the wall of the artery is the very short edge of the head and a short length of the sidewall and thus in conjunction with appropriate use of the device, thermal damage is held to desired levels. The maximum diameter of the device varies with intended use and typically with peripheral arteries may vary from 1 to 4 mm (.04 to .16 inch).

In both embodiments, the helically wound wire is brought out through a slot in the collar and is connected to a coaxial cable. A tube which carries a guide wire to the interior of the hollow cylindrical heater is encased, along with the coaxial cable, in an outer casing which, depending upon use, may or may not be enclosed within a spiral spring. An anchor wire is attached to the heater to prevent its detachment from the remainder of the catheter. The wire may be electrically conductive or coated with an electrically conductive material to prevent build-up of static electricity on this heater.

In use, entry is made into an artery that leads to the artery to be treated. A guide wire is inserted through the entry into the artery and while being observed by fluoroscopy is guided through the stenosis. Once the guide wire is satisfactorily located distal to the stenosis, the catheter is advanced over the guide wire until it reaches the stenosis. The coaxial cable is attached to an appropriate power supply, the power supply is turned on and the heater energized by magnetic flux produced by the coil when the power is applied, typically by means of a foot pedal switch.

The heater to be described herein is operated preferably at about 13.56 MHz and has an effective Curie temperature preferably in a range of 450°C to 620°C and perhaps as high as 700°C. The frequency of operation may vary depending upon size of the device, materials employed and the like and may vary from 10 MHz to 2 GHz. Specifically, the wattage of the power supply must be such as to cause the heater temperature to increase rapidly at lower temperatures and to have such high power operation as to rapidly re-establish desired temperature in the presence of a sudden increase in thermal load. Thus, the heater provides therapeutic ablation by vaporization with operating cycles as short as one second. Since the environment in which the various heaters will be employed varies with their use and thus a fixed figure is not possible, it appears that 18-42 watts at 13.56 MHz is sufficient at the power supply.

The invention will now be described, by way of example, with reference to the accompanying drawings, in which:

Figure 1 illustrates the core and tip of the catheter;
Figure 2 illustrates the collar employed in the catheter;
Figure 3 illustrates the tip and collar assembled;
Figure 4 illustrates the assembled tip and collar with the heater winding wound about the tip;
Figure 5 illustrates the completed tip, collar and winding assembly;
Figure 6 illustrates the head end, core and collar of the heater as the unitary structure of a second embodiment of the invention;
Figure 7 illustrates the core and head end of the heater of the catheter;
Figure 8 illustrates the collar which is fabricated as an integral part of the core and head but shown separately for purposes of clarity;
Figure 9 illustrates the assembled heater with the heater winding wound about the core;
Figure 10 illustrates in cross section the completed core, collar and potted winding;
Figure 11 illustrates an idealized temperature profile about the head of the heater of the catheter in a homogeneous medium;
Figure 12 illustrates the initial connections of the assembly of Figure 10 in a cable connected to the external components of the device;
Figure 13 illustrates the completed connection of the heater assembly to the cable;
Figure 14 illustrates the application of a spiral external spring to the assembly of Figure 13;
Figure 15 illustrates the members involved in the connections of the cable to the external components of the system employing the catheter;
Figure 16 comprises a series of graphs illustrating tissue ablated versus time for catheters of two different Curie temperatures, and

Figure 17 is a series of graphs illustrating ablation rate per unit energy as a function of axial force for a series of prototype catheters having various heater and overall diameters, the values of the No. 3 device approximating those of the device described in this application.

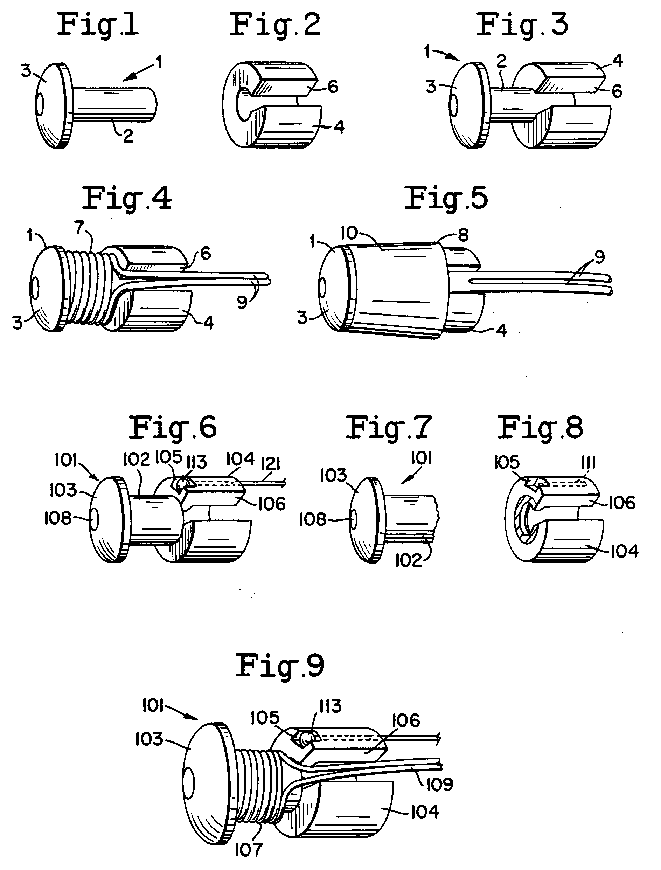

Referring now specifically to Figure 1 of the accompanying drawings, there is illustrated the hollow core and tip 1 of a catheter fabricated from high mu material, which term includes ferromagnetic, ferrimagnetic and like material. The core and tip include a hollow cylindrical body 2, the core, terminating at one end in an enlarged round head 3 coaxial with the cylindrical body 2. The center bore of the tip 1 should accommodate a 0.4 mm (.016 inch) guide wire.

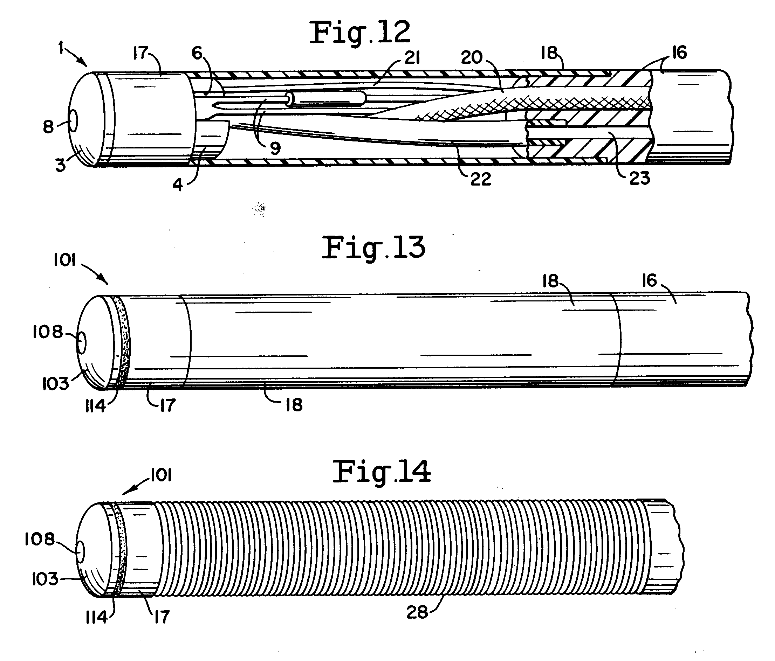

A collar 4 of low mu material is illustrated in Figure 2. The collar 4 is a hollow cylinder having a slot 6 extending parallel to the axis of the collar for purposes to be described. The interior dimension of the collar 4 is such as to snugly receive the end of the cylindrical body 2 to which it is secured, by welding for instance, by anchor wire 21; see Figure 12. A wire is wound about the body 2 to form a coil 7 with its ends 9 brought out through the slot 6 in the collar; Figure 4.

In one embodiment, the tip 1 is fabricated from a Type 430 stainless supplied by Carpenter Steel Division of Cartech. The collar 4 is fabricated also by the Carpenter Steel Division of Cartech. The material is a Type 304 stainless. The wire is 0.27% nickel clad high temperature copper wire.

Referring now to Figure 5 of the drawings, the space between the head 3 and the collar 4 is partially filled with a dielectric cross-over paste from Transene Company, Inc., Type 1500 which in turn is mixed with any one of several different types of bio-compatible glasses to provide a smooth transition 10 between the head and the collar 4. The inward taper of the head is roughly 5° from back to front. Some of the cross-over paste is disposed in the slot 6 of collar 4 to secure the wires in the slot. The specific materials currently employed are TGC-120 glass (a lead borosilicate glass) mixed 50/50 by weight with a dielectric cross-over paste (basically alumina powder mixed with cellulose) in a carbotol acetate solvent to render the material workable. The material is applied in layers and is baked in between to dry out the material. These materials may be replaced with comparable materials in the future. A tapered head is preferable but in some instances a non-tapered head may be employed.

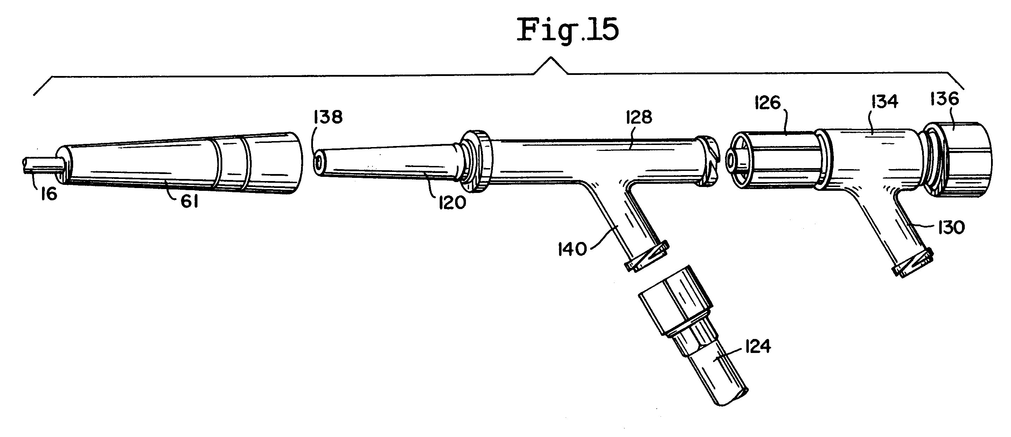

The connection of the catheter to the power supply and the remainder of the external equipment is described relative to Figures 12 and 15 hereof.

Referring now specifically to Figures 6-9 of the accompanying drawings, there is illustrated a second embodiment including a heater 101 of the catheter designed specifically to reduce heating of the side wall of the catheter. The heater is fabricated from material of high magnetic permeability, which term as used throughout includes ferromagnetic, ferrimagnetic and like material. The heater 101 includes a hollow cylindrical body 102 terminating at its distal end in an enlarged round, dome-shaped head 103 and at its proximate end in a collar 104, both coaxial with the cylindrical body 102. Center bore 108 of the heater 101 should accommodate a guide wire of not more than 0.4 mm (.016 inch) diameter.

The collar 104 is a unitary structure with the head and body 102; the head 103 and body 102 are illustrated separately in Figures 2 and 3, respectively, for purposes of clarity only. The collar 104 is cylindrical and has a slot 106 extending parallel to the axis of the collar for purposes to be described. The heater 101 may be secured, by welding, for instance, to an anchor wire 121, to prevent loss of the heater 101. Preferably, however, the collar has a rectangular recess 105 in its distal edge as viewed in Figures 1 and 2 communicating with a hole 111 extending axially through the remaining region of the collar so that an anchor wire 121 with a bulbous end 113 may be threaded through the hole with the bulbous end 113 seated in the recess 115. The anchor wire may have a thin coating of copper or other electrically conductive material to provide a conductive return to drain off static electricity. A wire of appropriate diameter (0.15mm or 34-40 AWG) is wound about the body 102 to form a coil 107 with its ends (wires) 109 brought out through the slot 106 in the collar; Figure 9.

In one embodiment, the heater 101, body 102, head 103 and collar 104 are fabricated from a Type 430 stainless supplied by Carpenter Steel Division of Cartech. The wire is 0.27% nickel clad high temperature copper wire having a ceramic coating for electrical insulation.

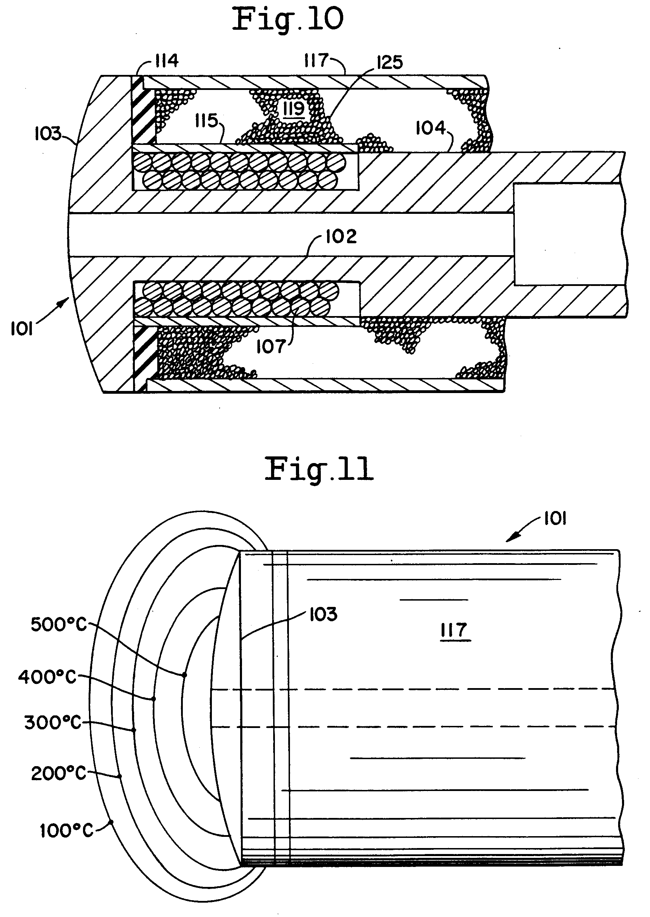

Referring now specifically to Figure 10, the coil 107 is wound to be abutting the head 103 and spaced from the collar 104 to cause flux to couple to and provide some direct heating in the head. Conversely the coil 107 is maintained a distance from the collar 104 to reduce coupling to and thus heating in the collar and thus along the side of the catheter.

The coil 107 is potted in a heat conductive material, for instance 50/50 mixture of an electrically non-conductive, heat conductive TGC-120 borosilica glass and Type 1500 cross-over-paste or other suitable material. The potted coil is surrounded by a sleeve 115 of stainless steel of the 400 series (preferably 430SS). The sleeve 115 has an internal diameter approximately equal to the diameter of the collar 104 and concentrates the flux in the region defined by the sleeve increasing efficiency, reducing flux coupling to the collar and also reducing radiation external to the catheter.

The combination of the placement of the coil 107, the use of heat conductive potting compound and sleeve 115 which is also heated by the flux developed by the coil, forms an axially thermally conductive structure that insures a concentration of heat in the head 103. An idealized heat pattern adjacent the head is illustrated in Figure 11 of the accompanying drawings.

A sleeve 117 of series 300 stainless steel (preferably 316SS), extends from the head 103 to a region radially aligned with the proximate end of the collar 107 remote from the head, but spaced radially outward from the collar 104. A thermally insulating body 114 of adhesive (S prime-mod from Zyp Coatings, Inc.) is disposed between sleeve 117 and head 103 to bind the sleeve to the head while providing some insulation of sleeve 117 from the adjacent head temperature. The sleeves 115 and 117 define a space 119 to maximize radial thermal resistance. The space 119 may be filled with microshells 125 that are fused together to provide a solid mass that excludes ambient air but which is 95% to 98% air. The microshells are available from the 3M Co. and others. Thus the only significant heating of the sleeve 117 is by conduction from the head 103 via body 114, which maintains the sleeve temperature below charring temperatures and below sticking temperatures except over a short enough length immediately adjacent the head that sticking does not occur.

Referring now specifically to Figure 12 of the accompanying drawings, there is illustrated the connections of the elements of the tip to various members that provide for connection to the external elements of the system such as the power supply, the guide wire insertion tube, etc. The only difference between the arrangement of Figure 12 and subsequently Figures 13-15 and that related to the first embodiment is the addition of the heat insulating ring 114 in Figures 13-15. One of the coil leads 9 is connected to the center conductor of a coaxial cable 20 that serves as a transmission line, while the other wire 9 or 109 is connected to the braid of the cable 20. A hollow tube 22 has one end aligned with axial passage 8 or 108 through the heater 1. The lumenal extension of this tube extends to the end of the cable structures as discussed relative to Figure 10 and is adapted to receive a guide wire which may pass through the tube 22 and into and through the passage 8 or 108 in the heater 1 or 101. Dye may also be injected into the artery through the tube 22 and passage 8 or 108. The coaxial cable 20 and a lumen extending from tube 22 extend through a flexible hollow tube (extrusion) 16, which may be polyurethane, to the proximal end of the instrument. The anchor wire 121 used to prevent loss of the distal end of the catheter and which may be used as a ground wire is secured to the collar 4 or 104 and extends through the extrusion 16 to a three-port-Y 136, see Figure 15, where it is anchored. The region between the proximal end of the core 2 or 102 and the extrusion 16 is potted, a typical potting material being a Type A medical, adhesive silicon. A PTFE sleeve 18 (Figure 13) extends from the proximal end of the heater (collar) to the distal shoulder of the extrusion 16 to complete construction thereof. If desired in a particular application, an elongated spiral spring 28 (Figure 14) may be disposed about the catheter from adjacent the collar 4 or 104 to a region of the extrusion 16.

At the end of the extrusion 16 remote from the catheter heater 1 or 101 three members are brought out.

Referring now to Figure 15, there is illustrated an exploded view of the connections to the extrusion 16 at the end remote from the heater 1 or 101. The extrusion 16 passes through a sleeve 61 of flexible material employed to protect the end of the extrusion. Secured inside of the sleeve is the three-port-Y internally of which the extrusion 16 terminates. The coaxial cable 20 is brought out through port 140 to a coaxial connector 124. Attached to a second port 128 of the three-port-Y 120 is a Tuohy-Borst Adapter 126 or the like is inserted in the end of port 128, the adapter 126 having a swivel 134 which permits a main body member 120 of the adapter to be rotated about the axis of the port 128. The main body member 120 has a central bore 138 communicating both through the swivel to the proximals end of the guide wire and with the lumen 23 of the catheter; the central bore being adapted to sealably receive the guide wire while allowing movement of the guide wire. The main body member 120 has a side arm 130 that communicates by the central bore 138 and is adapted to receive fluid which may contain a media for injection into the catheter and through the hole 8 or 108 in the heater and thus into the artery.

In use, a guide wire, if one is employed, is inserted into an artery via an introducer or a guiding catheter and guided to the location of and through the stenosis. The heater catheter is then threaded onto the wire at its proximal end external to the patient's body and slid through the introducer or guiding catheter and along the wire until it reaches the stenosis. The heater is brought into contact with the stenosis and is then activated to ablate the plaque to either create a passage through the plaque or to widen an existing passage whereby in either event to increase blood flow through the affected region. The procedure may, under appropriate circumstances, substantially clear the blockage or produce a passage large enough to permit adjunctive balloon angioplasty.

As previously indicated there are two main concerns relating to catheters employed primarily to remove atherosclerotic plaque from human arteries. The first concern relates to minimizing introduction of liquified plaque and/or charred pieces of vessel wall into the blood stream. The present device addresses these purposes by employing materials having high magnetic permeability that regulate at temperatures that ablate by vaporization the plaque so that it is absorbed into the blood in gaseous and fine particles form thus minimizing the dangers incident to its removal. Acceptable vaporization temperatures lie in the range of approximately 350°C to 500°C and specifically above 300°C below which temperature the catheter may stick to the walls of the artery and ablation rates are too low.

The second concern with the use of thermal catheters relates to damage to the walls of the artery due to excessive heat. By employing a structure where the heat is generated at the head 103 of the heater 101, and the core of the heater is surrounded with a coil, and the coil is surrounded with materials that are good heat conductors and a space is provided between the coil and outer sleeve, sidewall of the heater, the sides of the heater 101 are maintained except for a very short length at a temperature below that of the core and the head 103; the latter being a direct extension of the core. The heat employed to vaporize the plaque is primarily dissipated at the distal face 3 and is thus applied primarily to the plaque as opposed to the arterial walls. The present invention provides rapid reduction of temperature along sleeve 117 so as to minimize the length of the region of temperatures that can produce sticking. This region can be further reduced by placing a further sleeve in the heater to provide a region between this additional sleeve and sleeve 117 into which cooling fluid may be introduced. Cooling fluid could be circulated through a further port of the connector 136 containing input and output tubes connected to a fluid temperature control apparatus.

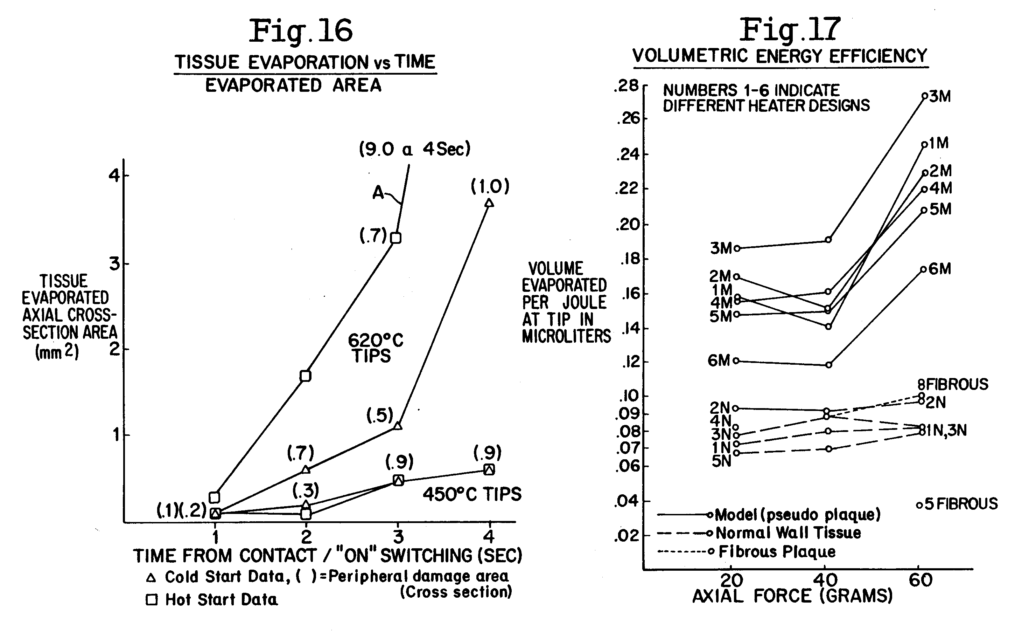

Referring specifically to Figure 16 of the accompanying drawings, there is illustrated a graph of the axial cross-sectional area of tissue ablation by the catheters of the present invention as a function of time, for heaters having Curie temperatures of 450°C and 620°C. It is readily apparent that the higher Curie temperature catheter vaporizes tissue at a far greater rate than the lower temperature catheter. The peripheral (wall) damage margin (cross-sectional area) is given in parentheses at each of the graph points. Damage for Graph A after 3 seconds is .7mm$^2$, an acceptable margin, roughly equivalent to that caused by a scalpel blade at the same depth.

It is apparent that the greater the power available to the heater, the faster it will rise to temperature and hold temperature in the face of changing, specifically, increasing load. The load varies greatly with the rate at which it is attempted to move the catheter through a blockage so that the more power that is available the more uniform the temperature will remain and the more quickly can the catheter be moved. As in any invasive procedure, it is most desirable to go in and get out as quickly as is commensurate with safe procedures and adequate reduction of stenosis.

In Figure 17 of the accompanying drawings the ablated volume is plotted as a function of axial force in grams for three different types of "blockage" materials. The numerical designations are: 1 and 2 discontinued models, 3 is an 2 mm (80 thousandth inch) outside diameter head, 4 is a 1.5 mm (60 thousandth) outside diameter head, 5 is a 1.8 mm 70 thousandth outside diameter head and 6 failed. The model (pseudo-plaque) is polyurethane gel of 90% water.

The term "constant current" as used herein refers to a current, I, where:

$$\frac{\Delta |I|}{I} < -1/2 \frac{\Delta |R|}{R}$$

The degree of self regulation is a function of the differences in degree of change of the two sides of the equation. If $\Delta |I|$ is equal to zero, good regulation is achieved as set forth in U.S. Patent No. 4,914,267. As the value of the left side of the equation approaches that of the right side, the degree of self regulation decreases. The power supply used may be any one of the supplies described in U.S. Patent No. 4,626,767; 4,752,864; 4,795,886 or 4,769,519 or other controlled current power supplies available on the market that can produce frequencies in the megacycle range. The first two supplies mentioned above by patent number operate at 13.56 MHz, preferably.

**Claims**

1. A thermal atherectomy device for use in blood vessels and the like comprising a tip (1, 101) including a cylindrical body (2, 102) terminating at one end in a head (3, 103), characterised in that the tip (1, 101) is of high magnetic permeability, in that the head (3, 103) is enlarged and the cylindrical body (2,102) terminates at its other end in an enlarged collar (4, 104), and, in that the device includes a coil (7, 107) of wire wound about the cylindrical body (2, 102) essentially abutting the head (3, 103) and removed from the collar (4, 104) and means (9, 20) for connecting the coil (7, 107) to a source of alternating current.

2. A device as claimed in Claim 1, wherein the means for connecting includes a coaxial cable (20) connected to the coil (7, 107) and connectable to a source of current, the cable (20) serving as a transmission line.

3. A device as claimed in Claim 1, wherein the connecting means comprises a transmission line (20) having a proximal end connected to a source of current signal and a distal end, the transmission line being constructed and arranged to pass through the interior of a body cavity.

4. A device as claimed in either Claim 2 or Claim 3, wherein the transmission line (20) is constructed and arranged to pass through a blood vessel so that the occlusive effect of plaque residing within the vessel may be reduced.

5. An interventional therapeutic device for remote delivery of heat to body tissue comprising an oscillator for providing an r.f. output signal within the range of 10 MHz to 2 GHZ, and, a thermal atherectomy device as claimed in any preceding Claim, the coil (7, 107) being connected to the oscillator and operating to convert the r.f. signal transmitted thereto into heating of the tip (1, 101), wherein the conversion is optimized at a predetermined frequency, the frequency of the output signal from the oscillator being set at substantially the predetermined frequency, whereby the tip (1, 101) is heated to a temperature sufficient for therapeutic effects.

6. A device as claimed in any preceding Claim, further comprising a high magnetic permeability cylindrical sleeve (115) disposed in close proximity about the coil (7, 107) and of less diameter than the head (3, 103), the coil (7, 107) being enclosed within a heat conductive potting compound within the sleeve (115).

7. A device as claimed in Claim 6, further comprising a non-magnetic sleeve (117) of a diameter of the head (3, 103) and extending between the head (3, 103) and the collar (4, 104) to define a space (119) between the sleeves (115, 117).

8. A device as claimed in any one of Claims 1 to 5, further comprising a non-magnetic sleeve (117) of about the diameter of the head (3, 103) and extending between the head (3, 103) and collar (4, 104) and means (114, 115, 119, 125) for maintaining all but a short length of the sleeve (117) adjacent the head (3, 103) below a temperature at which the sleeve (117) sticks to a wall of the blood vessel.

9. A device as claimed in Claim 8, wherein the means for maintaining comprises a sleeve (115) of high magnetic permeability dispersed about the coil (7, 107) and aligned with and of lesser diameter than the non-magnetic sleeve (117).

10. A device as claimed in either Claim 7 or Claim 9, further comprising a poor heat conductor (125) in the space (119) between the sleeves (115, 117).

11. A device as claimed in any one of Claims 7, 9 or 10, further including a poor heat conducting material (114) for securing the non-magnetic sleeve (117) to the head (3, 103).

12. A device as claimed in any preceding Claim, further having a smooth coating of low heat conductive material covering the coil (7, 107).

13. A device as claimed in Claim 12, wherein the coating comprises a mixture of a glass and a dielectric cross-over paste disposed over the coil (7, 107).

14. A device as claimed in any preceding Claim, wherein the tip (1, 101) has an axial passage therethrough to receive a guide wire.

15. A device according to Claim 14, further comprising a hollow tube (22) communicating with the passage.

16. A device as claimed in any preceding Claim, wherein the high permeability magnetic material has a Curie temperature above 400°C preferably in the range of 450°C to 700°C.

17. A device as claimed in any preceding Claim, further comprising means (121) for preventing static voltage build-up on the tip (1, 101).

18. A device as claimed in Claim 17, wherein the preventative means comprises an anchor wire (121) for the tip (1, 101).

19. A device as claimed in Claim 18, wherein the anchor wire (121) is secured to the collar (4, 104).

20. A device as claimed in any preceding Claim, wherein the collar (4, 104) has a slot (6, 106) through which the wires (9, 109) at the ends of the coil (7, 107) extend.

**Patentansprüche**

1. Vorrichtung zur thermischen Atherektomie zur Verwendung in Blutgefäßen und dergleichen, mit einer Spitze (1, 101), die einen zylindrischen Körper (2, 102) aufweist, der an einem Ende in einem Kopf (3, 103) endet, **dadurch gekennzeichnet,** daß die Spitze (1, 101) von hoher magnetischer Permeabilität ist, daß der Kopf (3, 103) vergrößert ist und der zylindrische Körper (2, 102) an seinem anderen Ende in einem vergrößerten Ring (4, 104) endet und daß die Vorrichtung eine Spule (7, 107) aus um den zylindrischen Körper (2, 102) gewickeltem Draht aufweist, die im wesentlichen an dem Kopf (3, 103) anliegt und von dem Ring (4, 104) entfernt ist, und wobei Mittel (9, 20) zur Verbindung der Spule (7, 107) mit einer Wechselstromquelle vorgesehen sind.

2. Vorrichtung nach Anspruch 1, wobei die Mittel zur Verbindung ein Koaxialkabel (20) aufweisen, das mit der Spule (7, 107) verbunden ist und mit einer Stromquelle verbindbar ist, wobei das Kabel (20) als Übertragungsleitung dient.

3. Vorrichtung nach Anspruch 1, wobei die Verbindungsmittel eine Übertragungsleitung (20) aufweisen, die ein proximales Ende, das mit einer Quelle eines Stromsignales verbunden ist, und ein distales Ende aufweist, wobei die Übertragungsleitung dafür konstruiert und eingerichtet ist, durch das Innere eines Körperhohlraumes hindurchzugehen.

4. Vorrichtung nach Anspruch 2 oder 3, bei der die Übertragungsleitung (20) dafür konstruiert und eingerichtet ist, durch ein Blutgefäß hindurchzugehen, so daß der verstopfende Effekt von Belag, der sich innerhalb des Gefäßes befindet, reduziert werden kann.

5. Therapeutische Eingriffs-Vorrichtung zur Fernabgabe von Wärme an Körpergewebe, mit einem Oszillator zum Erzeugen eines HF-Ausgangssignales innerhalb des Bereiches von 10 MHz bis 2 GHz und mit einer Vorrichtung zur thermischen Atherektomie gemäß einem der vorhergehenden Ansprüche, wobei die Spule (7, 107) mit dem Oszillator verbunden ist und arbeitet, um das dorthin übertragene HF-Signal in eine Erwärmung der Spitze (1, 101) umzusetzen, wobei die Umsetzung bei einer vorbestimmten Frequenz optimiert wird, wobei die Frequenz des Ausgangssignales von dem Oszillator bei im wesentlichen der vorbestimmten Frequenz eingestellt wird, wobei die Spitze (1, 101) auf eine Temperatur erwärmt wird, die für therapeutische Wirkungen ausreichend ist.

6. Vorrichtung nach einem der vorhergehenden Ansprüche, die ferner aufweist eine zylindrische Hülse (115) hoher magnetischer Permeabilität, die in unmittelbarer Nähe auf der Spule (7, 107) angeordnet ist und einen geringeren Durchmesser als der Kopf (3, 103) aufweist, wobei die Spule (7, 107) innerhalb einer wärmeleitenden Einbettungsmasse innerhalb der Hülse (115) eingeschlossen ist.

7. Vorrichtung nach Anspruch 6, die ferner eine nicht-magnetische Hülse (117) mit dem Durchmesser des Kopfes (3, 103) aufweist, die sich zwischen dem Kopf (3, 103) und dem Ring (4, 104) erstreckt, um einen Raum (119) zwischen den Hülsen (115, 117) zu bilden.

8. Vorrichtung nach einem der Ansprüche 1 bis 5, die ferner aufweist eine nicht-magnetische Hülse (117) von ungefähr dem Durchmesser des Kopfes (3, 103), die sich zwischen dem Kopf (3, 103) und dem Ring (4, 104) erstreckt, und Mittel (114, 115, 119, 125) zum Aufrechterhalten der gesamten Hülse (117) mit Ausnahme einer geringen Länge benachbart zu dem Kopf (3, 103) unterhalb einer Temperatur, bei der die Hülse (117) an einer Wandung des Blutgefäßes anhaftet.

9. Vorrichtung nach Anspruch 8, bei der die Mittel zum Aufrechterhalten eine Hülse (115) hoher magnetischer Permeabilität aufweisen, die um die Spule (7, 107) herum angeordnet ist und die mit der nicht-magnetischen Hülse (117) ausgerichtet ist und einen geringeren Durchmesser als diese aufweist.

10. Vorrichtung nach Anspruch 7 oder 9, die ferner einen schlechten Wärmeleiter (125) in dem Raum (119) zwischen den Hülsen (115, 117) aufweist.

11. Vorrichtung nach einem der Ansprüche 7, 9 oder 10, die ferner ein schlecht wärmeleitendes Material (114) zum Sichern der nicht-magnetischen Hülse (117) an dem Kopf (3, 103) aufweist.

12. Vorrichtung nach einem der vorhergehenden Ansprüche, die ferner eine feine Beschichtung eines niedrigwärmeleitfähigen Materials aufweist, die die Spule (7, 107) bedeckt.

13. Vorrichtung nach Anspruch 12, bei der die Beschichtung eine Mischung eines Glases und einer dielektrischen Verbindungsmasse aufweist, die über der Spule (7, 107) angeordnet ist.

14. Vorrichtung nach einem der vorhergehenden Ansprüche, bei der die Spitze (1, 101) einen axialen Durchgang durch die Spitze hindurch aufweist, um einen Führungsdraht aufzunehmen.

15. Vorrichtung nach Anspruch 14, die ferner ein hohles Rohr (22) aufweist, das mit dem Durchgang in Verbindung steht.

16. Vorrichtung nach einem der vorhergehenden Ansprüche, bei der das Material hoher magnetischer Permeabilität eine Curietemperatur oberhalb von 400°C, vorzugsweise in dem Bereich von 450°C bis 700°C, aufweist.

17. Vorrichtung nach einem der vorhergehenden Ansprüche, die ferner Mittel (121) zur Verhinderung einer statischen Aufladung an der Spitze (1, 101) aufweist.

18. Vorrichtung nach Anspruch 17, bei der die Verhinderungsmittel einen Verankerungsdraht (121) für die Spitze (1, 101) aufweisen.

19. Vorrichtung nach Anspruch 18, bei der der Verankerungsdraht (121) an dem Ring (4, 104) gesichert ist.

20. Vorrichtung nach einem der vorhergehenden Ansprüche, bei der der Ring (4, 104) einen Schlitz (6, 106) aufweist, durch den sich die Drähte (9, 109) an den Enden der Spule (7, 107) erstrecken.

## Revendications

1. Un dispositif d'athérectomie thermique, destiné à être utilisé dans des vaisseaux sanguins et analogues, comprenant un embout (1, 101), comprenant un corps cylindrique (2, 102) se terminant à une extrémité en une tête (3, 103), caractérisé en ce que l'embout (1, 101) est d'une perméabilité magnétique élevée, en ce que la tête (3, 103) est élargie et le corps cylindrique (2, 102) se termine à son autre extrémité en une collerette (4, 104) élargie, et en ce que le dispositif comprend une bobine (7, 107) en fil métallique enroulé autour du corps cylindrique (2, 102) venant sensiblement en butée avec la tête (3, 103) et dégagée de la collerette (4, 104), et des moyens (9, 20) pour assurer la connexion de la bobine (7, 107) à une source de courant alternatif.

2. Un dispositif selon la revendication 1, dans lequel les moyens de connexion comprennent un câble coaxial (20) connecté à la bobine (7, 107) et susceptible d'être connecté à une source de courant, le câble (20) servant de ligne de transmission.

3. Un dispositif selon la revendication 1, dans lequel le moyen de connexion comprend une ligne de transmission (20) ayant une extrémité proximale connectée à une source de signal de courant et une extrémité distale, la ligne de transmission étant construite et agencée pour passer par l'intérieur d'une cavité corporelle.

4. Un dispositif selon la revendication 2 ou la revendication 3, dans lequel la ligne de transmission (20) est construite et agencée pour passer par un vaisseau sanguin de manière que l'effet occlusif d'une plaque résidant dans le vaisseau puisse être réduit.

5. Un dispositif thérapeutique d'intervention destiné à fournir à distance de la chaleur à des tissus corporels, comprenant un oscillateur prévu pour fournir un signal de sortie de radiofréquence, dans la plage de 10 MHz à 2 GHz, et un dispositif d'athérectomie thermique tel que revendiqué dans l'une quelconque des revendications précédentes, la bobine (7, 107) étant connectée à l'oscillateur et fonctionnant pour convertir le signal de radiofréquence lui étant transmis en un chauffage de l'embout (1, 101), dans lequel la conversion est optimisée à une fréquence prédéterminée, la fréquence du signal de sortie venant de l'oscillateur étant fixée sensiblement à une fréquence prédéterminée, de manière que l'embout (1, 101) soit chauffé à une température suffisante pour produire des effets thérapeutiques.

6. Un dispositif selon l'une quelconque des revendications précédentes, comprenant en outre un manchon cylindrique (115) à perméabilité magnétique élevée et disposé à proximité immédiate autour de la bobine (7, 107) et ayant un diamètre inférieur à celui de la tête (3, 103), la bobine (7, 107) étant enfermée dans un composé d'enrobage conducteur de la chaleur, placé à l'intérieur du manchon (115).

7. Un dispositif selon la revendication 6, comprenant en outre un manchon (117) non magnétique, du diamètre de la tête (3, 103) et s'étendant entre la tête (3, 103) et la collerette (4, 104) afin de définir un espace (119) entre les manchons (115, 117).

8. Un dispositif selon l'une quelconque des revendications 1 à 5, comprenant en outre un manchon (117) non magnétique ayant un diamètre à peu près égal à celui de la tête (3, 103) et s'étendant entre la tête (3, 103) et la collerette (4, 104), et des moyens (114, 115, 119, 125) pour maintenir la totalité, mais sur une courte longueur du manchon (117) adjacent à la tête (3, 103), à une température inférieure à celle à laquelle le manchon (117) colle à une paroi du vaisseau sanguin.

9. Un dispositif selon la revendication 8, dans lequel le moyen de maintien comprend un manchon (115) ayant une perméabilité magnétique élevée, dispersé autour de la bobine (7, 107) et aligné avec le manchon non magnétique (117) tout en ayant un diamètre inférieur à celui-ci.

10. Un dispositif selon la revendication 7 ou la revendication 9, comprenant en outre un mauvais conducteur de la chaleur (125) placé dans l'espace (119) existant entre les manchons (115, 117).

11. Un dispositif selon l'une quelconque des revendications 7, 9 ou 10, comprenant en outre un matériau mauvais conducteur de la chaleur (114), prévu pour fixer le manchon non magnétique (117) à la tête (3, 103).

12. Un dispositif selon l'une quelconque des revendications précédentes, comprenant en outre un revêtement lisse réalisé en un matériau présentant une faible conductivité thermique et recouvrant la bobine (7, 107).

13. Un dispositif selon la revendication 12, dans lequel le revêtement comprend un mélange de verre et d'une pâte de jonction diélectrique disposée sur la bobine (7, 107).

14. Un dispositif selon l'une quelconque des revendications précédentes, dans lequel l'embout (1, 101) est traversé par un passage axial destiné à recevoir un fil de guidage.

15. Un dispositif selon la revendication 14, comprenant en outre un tube creux (22) communiquant avec le passage.

**16.** Un dispositif selon l'une quelconque des revendications précédentes, dans lequel le matériau magnétique à perméabilité élevée a une température de Curie supérieure à 400°C, de préférence dans la plage de 450 à 700°C.

**17.** Un dispositif selon l'une quelconque des revendications précédentes, comprenant en outre des moyens (121) pour empêcher tout établissement d'une tension statique sur l'embout (1, 101).

**18.** Un dispositif selon la revendication 17, dans lequel le moyen préventif comprend un fil d'ancrage (121) pour l'embout (1, 101).

**19.** Un dispositif selon la revendication 18, dans lequel le fil d'ancrage (121) est fixé à la collerette (4, 104).

**20.** Un dispositif selon l'une quelconque des revendications précédentes, dans lequel la collerette (4, 104) a une fente (6, 106) à travers laquelle s'étendent les fils (9, 109) s'étendent aux extrémités de la bobine (7, 107).

# Fig.1

# Fig.2

# Fig.3

# Fig.4

# Fig.5

# Fig.6

# Fig.7

# Fig.8

# Fig.9

# Fig.10

# Fig.11

Fig.12

Fig.13

Fig.14

14

Fig.15

# Fig.16

## TISSUE EVAPORATION vs TIME
### EVAPORATED AREA

TISSUE EVAPORATED AXIAL CROSS-SECTION AREA (mm²)

(9.0 a 4Sec)

A

(1.0)

(.7)

620°C TIPS

(.5)

(.7)

(.9)

(.9)

(.3)

450°C TIPS

(.1)(.2)

TIME FROM CONTACT / "ON" SWITCHING (SEC)

△ Cold Start Data, ( )=Peripheral damage area
□ Hot Start Data                (Cross section)

# Fig.17

## VOLUMETRIC ENERGY EFFICIENCY

VOLUME EVAPORATED PER JOULE AT TIP IN MICROLITERS

NUMBERS 1-6 INDICATE DIFFERENT HEATER DESIGNS

3M
1M
2M
4M
5M

3M

2M
1M
4M
5M

6M

6M

8FIBROUS
2N

2N
4N
3N
1N
5N

1N,3N

Model (pseudo plaque)           o 5 FIBROUS
Normal Wall Tissue
Fibrous Plaque

AXIAL FORCE (GRAMS)

EP 0 466 872 B1